# EUROPEAN PATENT APPLICATION

(11) **EP 1 029 547 A1**
(43) Date of publication of application: **23.08.2000**
(21) Application number: 99102962.0
(22) Date of filing: 15.02.1999
(51) Int. Cl.: A61K 38/55

(54) **Pharmaceutically active compounds and method for identifying same**

(71) Applicant: BOEHRINGER INGELHEIM INTERNATIONAL GmbH, 55218 Ingelheim am Rhein (DE)
(72) Inventor: Uhlmann, Frank, Dr., 1030 Wien (AT); Nasmyth, Kim, Dr., 1010 Wien (AT)
(74) Representative: Laudien, Dieter, Dr.

(57) **Abstract**

Pharmaceutically active compounds that interfere with or modulate sister chromatid separation by modulating the activity that cleaves one or more sister chromatid cohesion proteins, such as cohesin subunits, and method for identifying such compounds.

## Description

The invention relates to the field of therapy, in particular to the inhibition of the proliferation of rapidly diving cells.

During the process of cell division, sister chromatids are pulled to opposite halves of the cell by microtubules emanating from spindle poles at opposite sides of the cell. One set of microtubules inter-digitates with others emanating from the opposite pole. Their role is to keep (and drive) the two poles apart. Meanwhile, a second set of microtubules attaches to chromosomes via specialized structures called kinetochores and pulls them towards the poles. Sister chromatids segregate away from each other because their kinetochores attach to microtubules emanating from opposite poles (Rieder et al., 1998). Chromosomes are not mere passengers during this process. During metaphase, the tendency of microtubules to move sisters apart is counteracted by cohesion holding sisters together. Cohesion therefore generates the tension by which cells align sister chromatids on the metaphase plate. Were sisters to separate before spindle formation, it is difficult to imagine how cells could distinguish sisters from chromatids that were merely homologous. The sudden loss of cohesion, rather than an increase in the exertion of microtubules, is thought to trigger sister separation during anaphase (Miyazaki et al., 1994). Cohesion also prevents chromosomes falling apart due to double strand breaks and facilitates their repair using recombination.

To avoid missegregation of chromosomes, anaphase must only be initiated after sister chromatids of each duplicated chromosome have attached to opposite poles of the mitotic spindle. Microtubules emanating from opposing spindle poles attach to a specialised structure on each sister chromatid called the kinetochore. Microtubules are thought to "find" kinetochores by a "search and capture" mechanism which cannot be completed simultaneously for all chromosomes (Hayden et al., 1990; Merdes and De Mey, 1990). Cells therefore possess regulatory mechanisms that delay sister chromatid separation until the last chromosome has achieved bipolar attachment. The dissolution of sister chromatid cohesion at the metaphase to anaphase transition is therefore a highly regulated step during the eukaryotic cell cycle.

Sister chromatid cohesion depends on a multi-subunit complex called cohesin (Losoda et al., 1998), which contains at least four subunits: Smc1p, Smc3p, Scc1p, and Scc3p, all of which are conserved between yeast and humans. It is likely, but not yet proven, that cohesin is a key constituent of the tether that holds sister chromatids together. The association between cohesin and chromosomes has recently been shown to depend on the Scc2 (Mis4) protein. Cohesion is established during DNA replication (Uhlmann and Nasmyth, 1998). It has been recently shown that the Ecol (Ctf7) protein is required for the establishment of cohesion during S phase but unlike cohesin is not required to maintain cohesion during G2 and M phases. In yeast, cohesin remains tightly associated with chromosomes until metaphase; that is, it is present on chromosomes during their alignment during metaphase. In animal cells, however, the bulk of cohesin dissociates from chromosomes during prophase. It is unclear how much cohesin, if any, remains on chromosomes during metaphase. The nature of the link that holds sister chromatids together during metaphase in animal cells is therefore unclear. It could either involve a small fraction of cohesin that remains associated with chromosomes or some other protein complex.

In yeast, at least two of cohesin's subunits, Scc1p and Scc3p, suddenly disappear from chromosomes at precisely the point at which sister chromatids separate (Michaelis et al., 1997). This has led to the notion that a sudden change in the state of cohesin might trigger the onset of anaphase, at least in yeast. In Drosophila, the meiS332 protein, which binds to chromosomes during prometaphase, also disappears at the onset of anaphase. MeiS332 is required for sister chromatid cohesion during meiosis but not during mitosis (Moore et al., 1998; Kerrebrock et al., 1995). MeiS332 is probably not a cohesin subunit nor is it apparent whether homologous proteins exist in humans.

Both the dissociation of Scc1p from chromosomes and the separation of sister chromatids are dependent on a specialized sister separating protein (a separin) called Esp1p (Ciosk et al., 1998). Homologues of Esp1 exist in the fission yeast S. pombe, in Aspergillus nidulans, in the nematode worm C.elegans, in Xenopus laevis, and in man. This strongly suggests that Esp1-like separins have a fundamental role in chromosome segregation that is largely conserved between yeast and man. Espl is tightly bound by an inhibitory protein called Pdslp whose destruction shortly before the metaphase to anaphase transition is triggered by ubiquitination mediated by the anaphase promoting complex (APC) (Cohen-Fix et al., 1996). The APC is large multi-subunit complex, most of whose subunits are conserved between yeast and humans. Together with activator proteins called Cdc20p and Cdh1p, it mediates the ubiquitination and thereby destruction of many different cell cycle proteins, including anaphase inhibitors like Pds1 and mitotic cyclins. Pdsl destruction is mediated by a form of the APC bound by the activator Cdc20. This form is called APC^{Cdc20}. (For a review, see Peters, 1998)

Proteins with similar properties to Pds1 have been found in fission yeast (Cut2p), in Xenopus, and in humans. The APC is essential for sister chromatid separation in most if not all eukaryotic organisms. In yeast, it is clear that its main role in promoting sister separation is to destroy Pds1, which liberates Esp1 and allows it to destroy sister chromatid cohesion, possibly by destroying the physical links between sisters mediated by cohesin.

It was an object of the invention to further eluciate the mechanism of sister chromatid separation.

In particular, it was an object of the invention to understand the mechanism by which Esp1p mediates the dissocation of Scc1p from chromosomes in the budding yeast in order to take advantage of this mechanism by using it as a target in human therapy, in particular of cancer therapy.

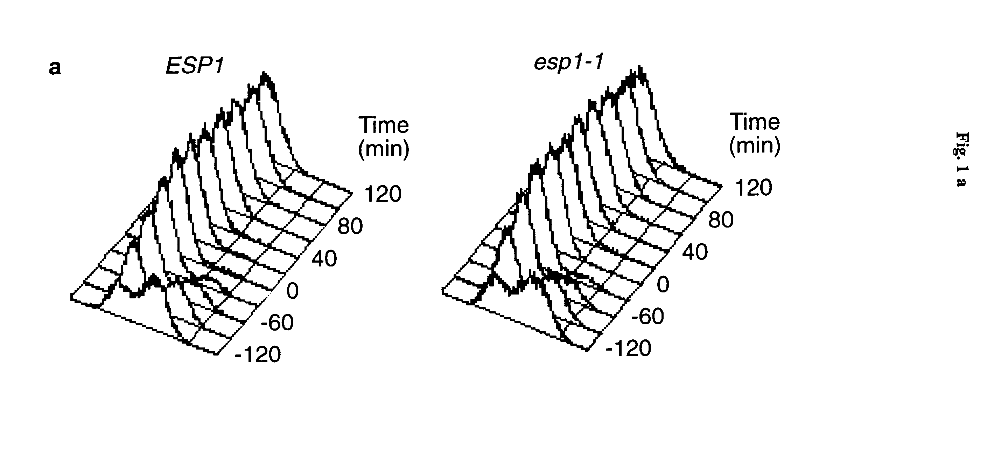

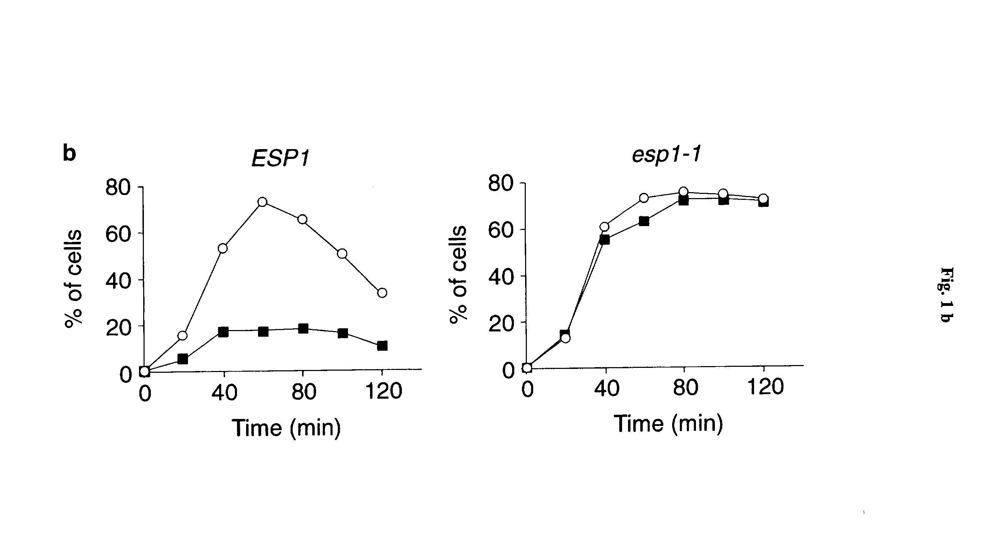

To solve the problem underlying the present invention, the following approach was taken: The observation that the dissociation of Scc1p from chromosomes at the onset of anaphase is dependent on Esp1 suggests that Esp1 might either have a direct role in this process. or that Esp1 might be indirectly involved by initiating a process that leads to Scc1p's dissociation. It was found in experiments of the present invention that Esp1 also prevents association of Scc1p with chromosomes during G1 (see Example 1), which strongly suggests that Esp1's role might be very direct. Scc1p is an unstable protein which is rapidly destroyed following its dissociation from chromosomes at the onset of anaphase and which must be re-synthesised during late G1 during the next cell cycle in order for cohesion to be established at the next round of DNA replication (Michaelis et al., 1997) It was found that Scc1p synthesised during G2 is also capable of binding to yeast chromosomes but that it fails to produce cohesion under these circumstances (Uhlmann and Nasmyth, 1998) However, it was noted that Scc1p synthesised during early G1 binds to chromosomes poorly, if at all. As shown in Figure 1, inactivation of Esp1 permits the efficient association between Scc1p and chromosomes during early G1. The implication is that Esp1 not only triggers Scc1p's dissocation from chromosomes at the onset of anaphase but also prevents Scc1p's stable association with chromosomes during the subsequent G1 period. This strongly suggests that Esp1 has a fairly direct role in controlling the association between Scc1p and chromosomes.

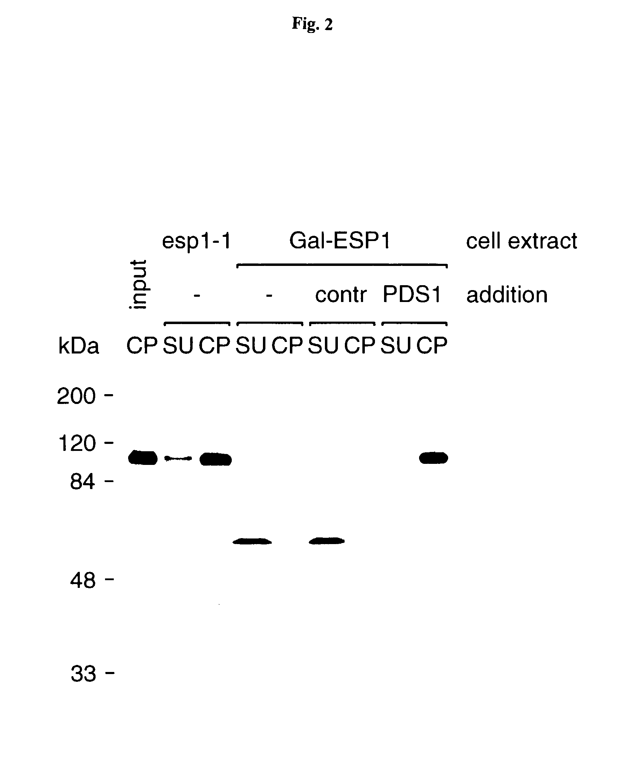

Starting from this finding, an assay was established by which Espl activity could be measured in vitro. A crude preparation of yeast chromatin isolated from cells arrested in a metaphase-like state by nocodazole, was incubated with a soluble extract prepared from cells over-producing Esp1 from the GAL promoter (Figure 2). To detect Scc1p cells were used whose Scc1 protein was tagged at its C-terminus with multiple HA or Myc epitopes, which can readily be detected with monoclonal antibodies. About 70% of the total Scc1p in nocodazole blocked cells is tightly associated with chromatin and is therefore present in the chromatin fraction that was used. Most of the Scc1p remains tightly associated with chromatin following incubation with an extract prepared from esp1-1 mutant cells but most disappears from the chromatin fraction upon incubation with extracts containing high levels of wild type Esp1 protein. Somewhat surprisingly, the Scc1p protein induced to dissociate from chromatin by Esp1 appeared in the "soluble" supernatant fraction as a cleaved product. The C-terminal fragments of this cleavage were detected by using as a substrate a C-terminally tagged Scc protein and N-terminal fragments using as substrate an N-terminally tagged Scc1 protein. The sizes of these cleavage products suggested that Esp1 induces one or more specific cleavages of Scc1p within a 10 kd interval. This Esp1-dependent cleavage was inhibited by the addition of reticulate lysate that had translated Pds1 but not by an otherwise identical lysate that had translated an unrelated control protein. The Esp1 activity detected by the cleavage assay is therefore inhibited by Pds1, which demonstrates directly, for the first time, that Pds1 is indeed an inhibitor of Esp1p.

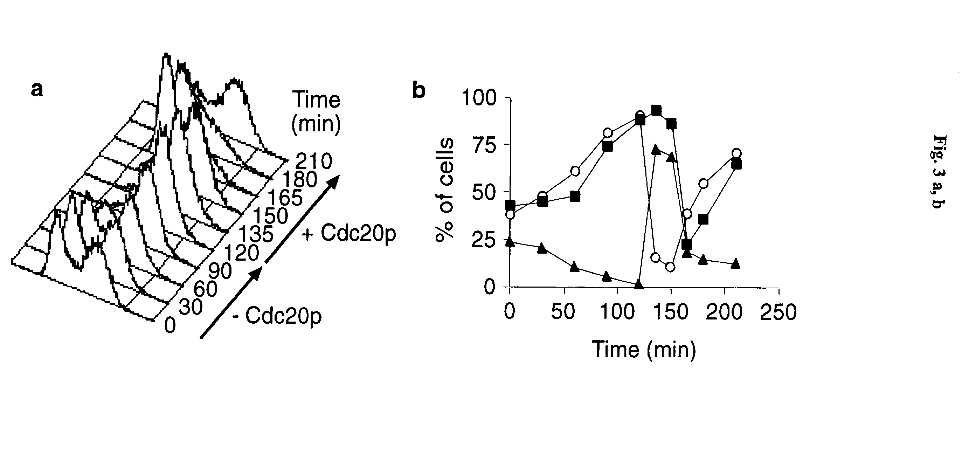

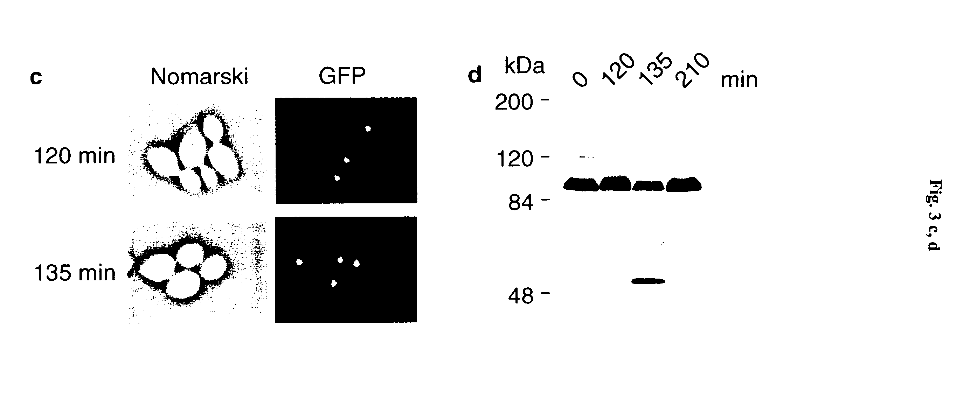

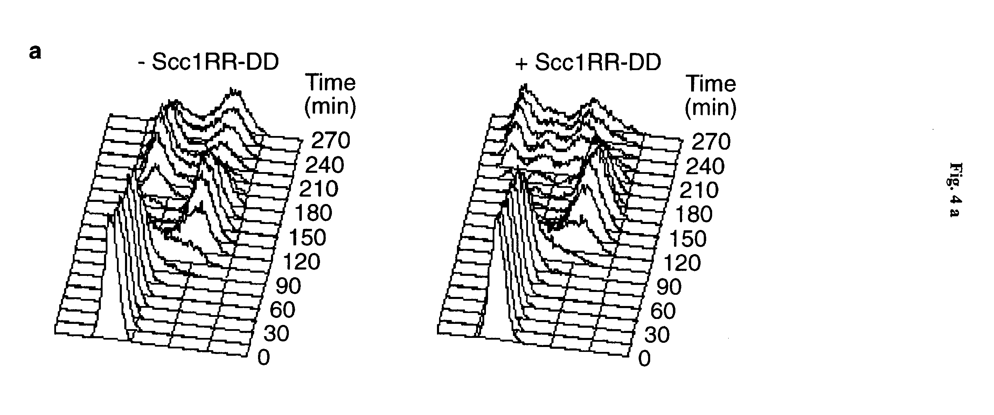

To address whether Esp1 induced cleavage of Scc1p also occurs in vivo at the onset of anaphase, a yeast strain was constructed in which expression of the APC activator Cdc20p is under control of the galactose inducible *GAL1-10* promoter. The strain also expressed an Scc1 protein tagged at its C-terminus with multiple HA or myc epitopes. Cells from this strain were arrested in metaphase by incubation in galactose free medium and then induced to embark on anaphase highly synchronously by the addition of galactose. Figure 3 shows that sister chromatids separate in most cells within 15 minutes and that Scc1p dissociates from chromosomes with similar if not identical kinetics. A low level of an Scc1 cleavage product was detected that is identical to that seen in vitro in cycling cells but none in cells arrested in metaphase. The cleavage product suddenly appeared upon induction with galactose with kinetics that were similar if not identical to the separation of sister chromatids and dissocation of Scc1p from chromosomes. To establish whether this in vivo cleavage was dependent on Esp1 activity the extent of Scc1p cleavage in wild type and esp1-1 mutants when released from cdc20 arrest at 35.5°C was compared (the restrictive temperature for esp1-1). The extent of Scc1p cleavage was greatly reduced in the esp1-1 mutant. It was concluded that Esp1 promotes the cleavage of Scc1p and its dissociation from chromosomes both in vivo and in vitro.

To address whether cleavage of Scc1 mediated by Esp1 is important either for sister chromatid separation or for Scc1p's dissociation from chromosomes, the cleavage site was mapped in order that it could then be mutated. An epitope tagged Scc1p protein from cells that had been stimulated to undergo anaphase by induction of Cdc20 expression was immunoprecipiated and the immunoprecipitated proteins were separated on SDS page. A short stretch of N-terminal amino acid sequence from the C-terminal cleavage fragment was then determined by Edman degradation. This showed that cleavage in vivo had occured between a pair of arginines at positions 268 and 269. The N-terminal of these arginine residues was then mutated to aspartic acid and an HA tagged version then was expressed from the *GAL1-10* promoter in yeast cells whose endogenous Scc1 protein was myc tagged. Galactose induced expression of this single mutant protein had little effect on cell proliferation. To establish whether the mutation had indeed abolished cleavage, chromatin from cells expressing the mutant protein was isolated and used as a substrate in the Esp1 assay. This showed that cleavage at site 268 was indeed eliminated by the aspartic acid mutation. However, the mutated protein was still cleaved in an Esp1-dependent manner. The C-terminal cleavage product from the mutant protein was about 10 kDa longer than that from wild type. The interpretation of these results is that Scc1p is actually cleaved at two sites approximately 10 kDa apart. Cleavage at the more C-terminal site is highly efficient, which is why C-terminal tagged proteins cleaved only at the more N-terminal site were rarely detected.

To identify the second cleavage site, sequences within Scc1p which are similar in sequence to those surrounding the known C-terminal cleavage site were looked for. A 5 out of 7 amino acid match at position 180 found. Furthermore, the distance between this potential site and the known cleavage site is consistent with the greater length of the cleavage product generated in vitro from protein whose C-terminal site (R268) had been mutated. The matching sequence also contained a pair of arginines and therefore the more N-terminal arginine was mutated to aspartic acid. Next the effect of expressing HA tagged versions of wild type Scc1p, both single mutant proteins, and the double mutant protein from the GAL promoter in yeast was compared. As a host for these studies a strain was used whose endogenous Scc1p was myc tagged. Neither wild type nor either single mutant blocked cell proliferation but expression of the double mutant protein was lethal. Chromatin from cells transiently expressing these proteins was prepared and it was shown that HA tagged double mutant protein was no longer cleaved when incubated in Esp1-containing extracts while the myc tagged wild type protein was efficiently cleaved.

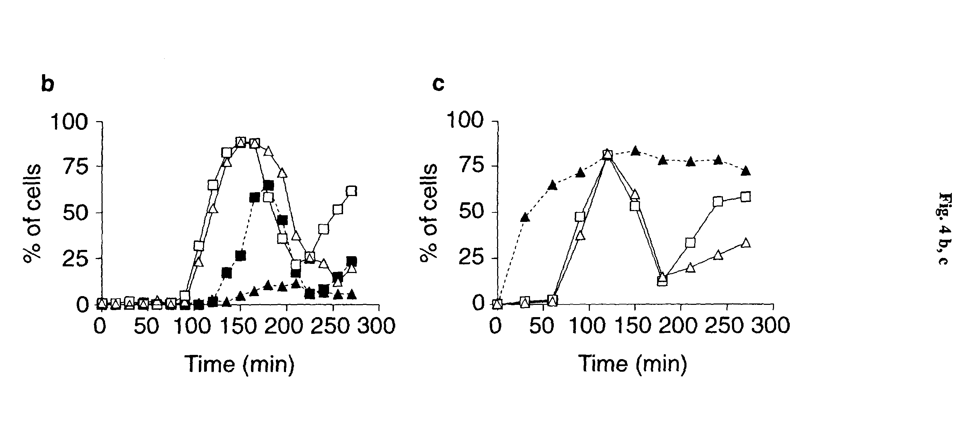

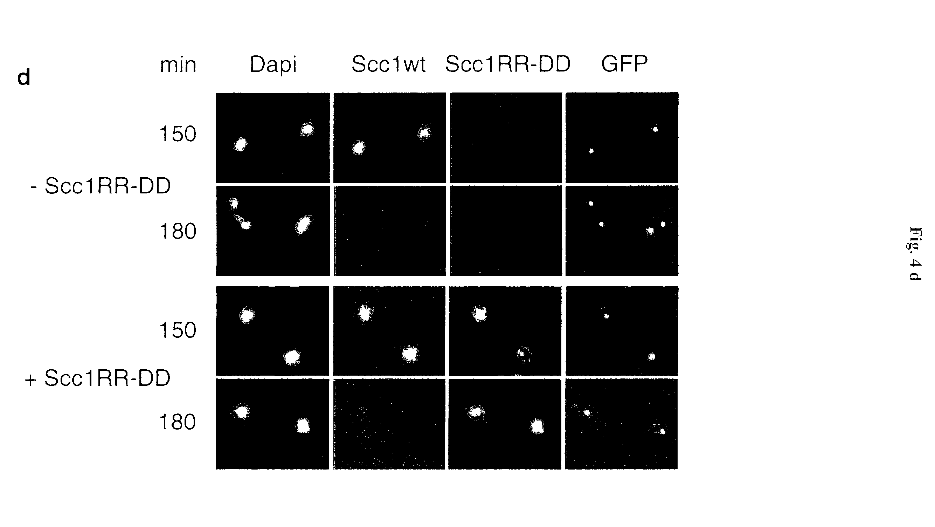

To investigate why cells expressing a non-cleavable Scc1p protein (the R180-D R268-D double mutant) cannot proliferate, centrifugal elutriation was used to isolate G1 cells from a culture growing in the absence of galactose , which were then incubated in the presence and absence of galactose (Fig. 4). In order to minimize the duration of mutant protein expression, the cells grown in the presence of galactose were transferred to glucose containing medium after most cells had replicated their DNA (at 135 min.). In the absence of galactose, sister separation and dissociation from chromosomes of endogenous myc tagged Scc1p occured simultaneously, approximately 60 min after DNA replication. Transient expression of double mutant protein greatly reduced sister chromatid separation (Fig. 4b) but did not affect dissociation of endogenous myc tagged wild type protein (Fig. 4c and d). Furthermore, the mutant protein remained tightly associated with chromosomes long after the endogenous wild type protein had disappeared. Expression of the mutant protein did not greatly delay cell cycle progression and most cells underwent cytokinesis, producing progeny with low (0-0.5C) amounts of DNA and cells with less than a 2C DNA content (Fig. 4a). The dissociation from chromosomes of wild type protein on schedule shows that the lack of sister separation in cells expressing non-cleavable Scc1p is not due to a lack of Esp1 activity. Collectively, the data obtained imply that cleavage of Scc1p at one of two sites is necessary both for sister chromatid separation and for dissociation of Scc1p from chromosomes.

From the obtained results it can be concluded that cohesin directly mediates the link between sister chromatids that is established during DNA replication and is maintained until metaphase. It can be further concluded that Esp1's activation by proteolysis of Pds1 (and by as yet to be identified other mechanisms) generates an activity inside cells that cleaves the Scc1p subunit of cohesin and that this event both destroys sister chromatid cohesion and causes Scc1p and possibly other cohesin subunits to dissociate from chromosomes.

The findings of the experiments of the present invention have shed the first key insight into the molecular mechanism by which eukaryotic cells separate sister chromatids. In view of the published literature, which contains no hints as to the mechanism by which sister chromatids are separated, the finding that an Esp1-related proteolytic activity is one of the key mechanisms involved in the onset of anaphase is highly surprising.

Given the high degree of conservation of these mechanisms in eucaryotic organisms, it can be concluded that the findings obtained in the experiments of the present invention in yeast are also valid in higher eucaryotic organisms, in particular in man.

Hence, interfering with this mechanism provides a novel approach for inhibiting the proliferation of rapidly diving animal cells, in particular tumor cells.

The invention relates to pharmaceutically active compounds which are characterized in that they interfere with or modulate sister chromatid separation by modulating the activity that cleaves one or more sister chromatid cohesion proteins, such as cohesin subunits.

(In the following, the proteolytic activity responsible for cleaving cohesin protein(s) and thus interfering with sister chromatid segregation, is termed "Espl-related activity".)

The pharmaceutically active compounds of the invention may be small chemical molecules or biological molecules, e.g. peptides.

In a first embodiment, the compounds of the invention are protease inhibitors specific for the protease that cleaves one or more sister chromatid cohesion proteins.

The compounds of the invention can be identified by the following methods:

In a first embodiment, the assay of the type described in Example 2 can be used to screen for compounds that inhibit the Esp1-related activity, i.e. Esp1- dependent cleavage of yeast Scc1p; this approach has the advantage that it is not necessary to know whether Esp1 is itself the protease responsible for cleavage of Scc1p. By this means, it is possible to identify compounds that inhibit Esp1-dependent cleavage of Scc1p. The efficacy of such compounds can then be tested for in vivo efficacy either on yeast cells or mammalian cells. Effective compounds should block (or at least in some way interfere with) sister chromatid separation, which can be measured (e.g. using CenV-GFP in yeast or standard cytological techniques in mammalian cells). Effective compounds should be either cytostatic (i.e. prevent cell division) or cytotoxic (e.g. cause apoptosis).

The interpretation of the data obtained in the experiments of the present invention suggests that Esp1 may itself be the protease responsible for the cleavage of Scc1p and/or other cohesin subunits. In this case, the compounds of the invention exert their effect by directly inhibiting Esp1's proteolytic activity, i.e. they are protease inhibitors specific for Esp1.

Therefore, in a next step, besides identifying a simpler substrate, the identity of the Esp1-dependent protease is determined in order to generate a more efficient assay suitable for high-throughput screening. This step may, for example, be carried out as follows:

It is first determined whether the Scc1 polypeptide alone or peptide sequences derived from it can act as substrates for the Esp1-related activity. First, radiolabelled Scc1 protein generated using in vitro translation of Scc1 mRNA (e.g. in a rabbit reticulocyte lysate) is added to yeast extracts containing high levels of Esp1 and cleavage monitored (e.g.by SDS PAGE followed by autoradiography). Second, defined peptides composed of amino acid sequences surrounding either the Scc1 cleavage sites (R180 and R268) are used as substrates. Cleavage of these peptides can be monitored by standard methods, e.g. by thin layer chromatography, by gel electrophoresis, by HPLC, or by mass spectroscopy. Third, fluorescent or other measurable derivatives of such peptides are generated and their cleavage in an Esp1-dependent manner is monitored by changes in the appropriate marker.

In case that substrates composed of Scc1p alone or peptides derived thereof prove not to be substrates for the Esp1-related activity, a yeast cohesin complex assembled from its constituent subunits can be tested for its suitability as a substrate. To this end, each subunit is recombinantly produced in large amounts, for example in yeast, or in E.coli, or in insect cells, then the subunits are allowed to assemble; a process that may take place either during or after their synthesis/purification. The reconstituted cohesin complex is then treated with yeast extracts prepared from cells over-producing Esp1 and cleavage of Scc1p is monitored, e.g. by SDS PAGE.

From the results obtained in the experiments of the invention, it may, inter alia, be concluded that Scc1p is the only cohesion protein cleaved as a consequence of Esp1-related activity or . Alternatively, other types of proteins, for example, those which regulate mitotic spindles, might also be targets/substrates of the Esp1-related activity . One way of addressing this question is to make a version of Scc1p that has one cleavage site replaced with a site for a foreign protease (with the other cleavage site removed). An example for a convenient protease to use is enterokinase, which has a very specific cleavage site (Asp-Asp-Asp-Asp-Lys). A strain can be constructed that contains: the SCC1 gene containing an enterokinase cleavage site, a chromosomal *cdc20-3* mutation, and the enterokinase gene under *GAL1-10* inducible control. In the presence of galactose at the restrictive temperature (when cdc20--3 cells are arrested in metaphase due to their failure to destroy Pds1), the effect of the artificial cleavage of Scc1p on its removal from chromosomes can be assayed (as measured by its presence in sedimented chromosomal DNA fractions). Whether or not this is sufficient for sister chromatid separation can also be examined microscopically, using the CenV-GFP system (see Fig. 3). These experiments allow to determine whether the rest of mitosis can proceed under these conditions in the absence of Esp1p function (note that Esp1 is inactive in cdc20-3 mutants at the restrictive temperature due to the presence of its inhibitor Pds1). If the foreign protease triggers Scc1p's dissociation from chromatids under these circumstances and sister chromatids segregate to opposite poles of the yeast cell, it can be concluded that cleavage of Scc1 is the sole function of Esp1 needed for sister chromatid segregation. If however, sister chromatids fail to segregate to opposite poles of the cell despite the variant Scc1p having been removed from chromatin, then is concluded that Esp1 has one or more functions besides cleavage of Scc1p. A clue as to these functions can be obtained from the phenotype of these cells and this can be used to identify other potential substrates for Esp1. Other substrates for Esp1 can be sought by looking for small DNA fragments from the yeast genome or an oligonucleotide library that can replace the normal Scc1 cleavage sites. Oligonucleotides may be inserted into a SCC1 gene (lacking both natural cleavage sites) under control of the GAL promoter on centromeric plamid. Yeast cells may be transformed with a library of such constructs and only plasmids whose modified Scc1 protein can be cleaved by the Esp1-related activiity will permit growth in the presence of galactose.

Esp1p can be produced recombinantly according to standard methods, e.g in yeast or insect cells or in other suitable host cells . Esp1p can be purified either by conventional biochemical fractionation from yeast cells over-producing Esp1 or by tagging the over-produced protein with polypeptide sequences which have a special affinity for a defined ligand (affinity purification). For example, Esp1 can be purified on nickel-agarose columns if it has been tagged with multiple histidine residues, whereas it can be purified on glutathione-agarose columns if it has been tagged with GST. Such affinity purification involves the cleavage of Esp1 from its tag using site specific proteases or self cleaving inteins. The thus obtained recombinant protein is then used to determine, according to known methods for assaying proteolytic activity, whether Esp1p alone is capable of cleaving Scc1p or peptide substrates derived from it.

In case that Esp 1 is alone capable of cleaving a peptide substrate derived from Scc1p, then an assay based on Esp1 as the protease and Sccp1 (peptide) as the substrate can easily be adapted to a high throughput format by methods that are standard for other defined proteases, as described below.

In the case that purified Esp1 alone does not catalyse the cleavage reaction, then this implies either that Esp1 is not itself the protease or that it requires a co-factor. In this case, Esp1 is indirectly involved in the proteolytic cleavage, e.g. by activating a protease, which might sit on chromosomes or by inducing self cleavage of Scc1p and/or other cohesion proteins. Esp1 may, for example, be an allosteric activator of the real protease.

Whether and by which mechanism Esp1 exerts an indirect effect on the cleavage of one or more cohesin subunits can be determined as follows:

The protease and/or its co-factor can be identified either by biochemical fractionation and/or by genetic approaches. In the first instance, cleavage of Scc1 can be detected when pure Esp 1 protein is added to yeast extracts lacking any Esp 1 activity. The factor(s) from the yeast extracts which enable the pure Esp1 to promote cleavage of Scc1 can then be purified by conventional biochemical techniques (e.g. fractionation) and identified by e.g. mass spectrometry. An alternative approach to identifying factors besides Esp1 needed for Scc1 cleavage is the use of yeast genetics. Mutations that reduce the activity of the protease or cofactor may be expected to be synthetic lethal either with expression of Scc1 mutants lacking one of its two cleavage sites or with esp1-1 mutants. Searching for such synthetic lethal mutations leads to the isolation of the putative cofactor and/or protease genes. Two types of yeast strains can be used for these genetic screens. In the first instance, a strain may be used that expresses from the galactose inducible GAL1-10 promoter a mutant Scc1 protein one of whose cleavage sites has been eliminated. This strain can be mutagenized, e.g. with EMS, and colonies grown on plates lacking galactose. Such colonies are then replica plated onto plates containing galactose and scored for growth by visual inspection. By this means, mutations in genes other than SCC1, which are incompatible with expression of a partially protease resistant Scc1 protein, can be identified. Assuming that the mutations are recessive to wild type, the wild type version of the mutant genes can be isolated by complementation of the lack of growth on galactose plates. The gene(s) can be identified by DNA sequencing.

In the second instance, a yeast strain can be generated whose endogenous ESP1 gene carries an esp1 mutation (e.g. esp1-1) and which carries an unstable centromeric plasmid carrying a colony colour marker (e.g. ADE3) and a wild type copy of ESP 1. This strain may also harbour mutations of ade2 and ade3. Colonies carrying the centromeric ESP 1 ADE3 plasmid will have a red colour, whereas colonies in which the plasmid has been lost will be white. The starting strain for mutagenesis, which is capable of losing the plasmid when grown at 23°C, will therefore produce red-white sectored colonies. Mutations that render this strain dependent on a wild type copy of ESP1 will no longer produce white sectors; that is, they will produce pure red colonies. The genes mutated in such strains can be identified by standard procedures, e.g.complementation of the mutant phenotype by centromeric plasmids carrying fragments from the yeast genome. By this means, genes can be identified whose products cooperate with Esp1 to cleave Scc1p can be identified. If Esp1 is not the protease, then the actual protease can be revealed by this method. Both genetic methods can also be used to identify putative co-factors in the cleavage process that are neither Esp1 nor a different protease itself.

The Esp1-dependent protease may also be identified by synthesising a peptide spanning the cleavage site (which is sufficient to confer cleavage) ending at its C-terminus with an aldehyde group. Such peptides are known in some cases to bind covalently to the catalytic site of proteases. Thus, a biotinylated version of such a peptide may label the protease so that it can be detected and purified using streptavidin.

Inhibitory compounds of this Esp1-related activity can be identified using a variation of the assays already described for the case that Esp1 itself is the protease. Instead of incubating peptide substrates with Esp1 alone, they can be incubated with a mixture of Esp1p, the protease, and its co-factors. All components can be purified by the methods outlined above for Esp1.

Given the existence of Esp1 homologues in man, it can be concluded that the Esp1-related activity plays an important role in triggering anaphase onset also in humans. An Esp1-dependent cleavage assay using human instead of yeast components can be established using the methods outlined above for yeast components.

ESTs and complete cDNA sequences for human Esp1 already exist in public databases (accession number KIAA0165). A human Esp1 cDNA can be tagged with sequences that permit its rapid purification and expressed at high levels, for example in E.coli, yeast, insect or mammalian cell cultures. If the yeast studies described above identify a separate protease and cofactors, then the existence of equivalent human genes can be confirmed first by inspection of existing databases.In the case of negative results, it is possible to identify the human genes by complementation of yeast mutants with human cDNAs; that is, a human cDNA library under control of a yeast promoter can be transformed into yeast mutants and clones are sought that rescue the mutant phenotype. A third possibility is to establish an assay for a human Esp1-related activity along the lines already successful in yeast; that is, cleavage of an appropriate substrate by human cell extracts containing high levels of human Esp1p can be assayed. To do this, the human ESP1 cDNA is expressed under control of a regulatable promoter in a mammalian cell line. Factors other than Esp1 1 required for the cleavage reaction can be purified by conventional biochemical fractionation and identified as outlined above for equivalent yeast proteins.

In the first instance, the human Esp1-related activity can be assayed using substrates developed to assay the equivalent yeast activity. In case the human protease has a different sequence specificity to the yeast protease, variants of the yeast peptide sequence can be systematically explored for suitable substrates. A large library of peptides can be generated by conventional means and this can be screened for peptides that are efficiently cleaved in an Esp1 dependent fashion either by pure human Esp1 or extracts containing large amounts of human Esp1. The cleavage assay may be performed as described above. Alternatively, the yeast sequences surrounding one of yeast Scc1p's cleavage sites can be replaced by variants of this site and the variants can be screened for their ability to be cleaved by human Esp1p when present at high levels in either yeast or human extracts. One way of enhancing the activity of human Esp1 for in vitro assays is to co-express it along with human Pds1 and then make extracts from cells in which Pds1 has just been destroyed by the APC. A third approach to identifying suitable substrates for a human Esp1-related activity is to screen human homologues of yeast cohesion proteins for their ability to be cleaved. Human homologues of all four known (and yet to be identified) yeast cohesin subunits are tested. In case the cohesion proteins cannot be cleaved, other proteins implicated in sister chromatid cohesion, in yeast (e.g. Pds5, Trf4) or animal cells (e.g. MeiS322 from Drosophila) can be tested.

Based on information about the sequence specificity of the proteolytic cleavage site in yeast, other potential substrates for the protease can be found in sequence data bases. This may form the basis for finding substrates in other organisms, including humans, and allows for the design of peptides which are useful as substrates in the screening assay of the invention. When developing the screening assay of the invention, which preferably comprises components of human origin, from yeast to human, it may be useful or necessary to use an intermediate assay based on the protease and/or its substrate derived from intermediate organisms. Such an intermediate assay can be based on cmponents from the fission yeast S. pombe. For example, the S.pombe homologue of Scc1 (called Rad21) contains two sequences, which are similar to the two known cleavage sites in Scc1 and Rad21 derived sequences may therefore be used to generate a substrate for S. pombe Esp1 (called Cut1). This process of advancing to higher organisms can be applied stepwise until a human system is attained. The cleavage site of any new substrate for human Esp1 can be determined by purifying the cleavage product and determining the N-terminal sequences by Edman degradation as described above.

The identification of the protease responsible for Esp1-related activity and the identification of potential co-factors, together with the identification of the substrate(s) for the protease and/or its co-factor(s), as described above, is the prerequisite for designing an assay that allows for the identification of substances interfering with sister chromatid separation.

In another aspect, the present invention relates to a method for identifying compounds that inhibit the proliferation of rapidly dividing cells, characterized in that the protease which is responsible for the proteolytic activity involved in sister chromatid separation, optionally together with one or more co-factors, is incubated, in the presence of the substrate(s) for the protease and optionally its co-factor(s), with test compounds and that the modulating effect of the test compounds on said proteolytic activity is determined.

This screening method of the invention is based on the assays for identifying inhibitors of the Esp1-related activitiy; which are described above, it may be performed with yeast constituents as assay components, and subsequently developed further stepwise using intermediate substrates, e.g. from S. pombe or Xenopus laevis, and finally equivalent human substrates (as described above).

The major components of the assay, i.e. the protease, its potential co-factors and the substrates, have been described above; the protease substrates useful in the above assay may be those equivalent to or mimicking the naturally occuring substrates, e.g. crude chromatin preparations, reconstituted cohesin complexes, or cohesion proteins like Scc1.

In a preferred embodiment, the substrate is a peptide containing the cleavage site of the naturally occuring substrate. The sequence specificity of the proteolytic cleavage can be determined by testing a variety of different peptides. These variants can be generated either by synthesising variant peptides or by mutating DNA sequences from genes encoding cohesion proteins (as described above).

The screening method of the invention to identify drug candidates for cancer therapy is carried out according to assay principles known in the art for identifying protease inhibitors. Such assays are based on the detection of the cleavage products of the substrate. To achieve this, a peptide or protein substrate that contains cleavage sites for the protease to be inhibited, is derivatized with a detectable lable, e.g a radioactive or a fluorescent label. Upon cleavage of the substrate by the protease, the cleavage product can be measured. If a test substance is an inhibitor of the protease, there is no detectable signal.

Recently, various assay methods for identifying protease inhibitors have been described that are amenable to automation in a high-throughput format, e.g. the method described by Cerretani et al., 1999, for hepatitis C virus NS3 protease, the method described by Ambrose et al., 1998, the microtiter colorimetric assay fot the HIV-1 protease described by Stebbins and Debouck, 1997, the fluorescence polarization assay described by Levine et al., 1997, the assay for hepatits C protease using quenched fluorogenic substrates described by Taliani et al., 1996, the method using immobilized peptide substrates described by Singh et al., 1996, the assay used for studying the inhibition of cathepsin G, using biotinylated and cysteine-modified peptides described by Brown et al., 1994. Additional examples for assays that may be used in the present invention for a high-throughput screening method to identify inhibitors of the Espl-related proteolytic activity were described by Gray et al., 1994, Murray et al., 1993, Sarubbi et al., 1991.

The compounds identified in the screening process of the invention are candidates for drugs that interfere with or modify the process of sister chromatid separation. Such drugs can either be used as anti-proliferative agents to combat proliferative diseases, in particular cancer. They can also be used to suppress instances whether premature sister separation causes pathology, for example in the origins of human aneuploidy.

For therapeutic use, candidate substances identified in the screening methods of the invention are further characterized in a secondary screen, as mentioned above, e.g. by testing their efficacy either on yeast cells or mammalian cells. Effective compounds should block sister chromatid separation, which can be measured in yeast or standard cytological techniques in mammalian cells. Effective compounds are either cytostatic or cytotoxic. Substances whose potential for therapeutic use has been confirmed in such secondary screen, are formulated and characterised in more detail for the development of medication in the usual way with regard to their pharmacological characteristics (bioavailibility, side effects, etc.), e.g. in suitable animal tumor models, and subsequently in clinical trials. Methods for the formulation of pharmaceutical preparations can be found in standard text books, e.g. Remington's Pharmaceutical Sciences, 1980.

Brief description of the figures:
- Fig. 1:: Chromosome association of Scc1p in G1 is Espl-dependent
- Fig. 2:: In vitro assay for Scc1p cleavage and dissociation from chromatin
- Fig. 3:: Detection of the Scc1p cleavage product in vivo in cells passing sychronously throught the metaphase to anaphase transition
- Fig. 4:: Expression of a non-cleavable variant of Scc1p prevents Scc1p dissociation from chromosomes and sister chromatid separation in vivo

If not otherwise stated, the following materials and methods were used in the experiments of the present invention

### a) Plasmids and strains:

The Scc1p coding sequence (Saccharomyces Genome Database YDL003W) was cloned under control of the *GAL1-10* promoter in a YIplacl28 derived vector (Gietz and Sugino, 1988), and under its own promoter into YCplac111 (Gietz and Sugino, 1988) using the polymerase chain reaction (PCR). DNA fragments encoding multiple HA and myc epitopes were inserted into restriction sites introduced by PCR at the N- and C-termini of SCC1. Site directed mutagenesis was performed by PCR using primers containing the desired nucleotide changes. The validity of all constructs was verified by nucleotide sequencing.

All strains used were derivatives of W303 *(HMLa HMRa ho ade2-1 trp1-1 can1-100 leu2-3,112 his3-11,15 ura3*). Epitope tags at the endogenous Scc1p were generated by a PCR one-step tagging method (Michaelis et al., 1997). A strain overexpressing Esp1p from the *GAL1-10* promoter was described (Ciosk et al., 1998) and crossed to a strain containing the *esp1-1* mutation (McGrew et al., 1992). A strain expressing the sole source of Cdc20p under control of the *GAL1-10* promoter was described in (Lim et al., 1998). To visualize sister chromatids a Tet repressor-GFP fusion protein is synthesized in the cells that binds to a cluster of Tet operator sequences integrated at the *URA3* locus close to the centromere of chromosome V as described in (Michaelis et al., 1997).

### b) Cell growth and cell cycle experiments

Cells were grown in complete medium (Rose et al., 1990) at 25°C if not otherwise stated. Strains expressing Cdc20p, Esp1p, or Scc1p from the *GAL1-10* promoter were grown in complete medium containing 2% Raffinose as carbon source. The *GAL1-10* promoter was induced by adding 2% Galactose. A G1 like arrest was achieved by adding 1 µg/ml of the pheromone alpha factor to the medium. For a metaphase arrest, 15 µg/ml nocodazole was added with 1% DMSO. Metaphase arrest due to Cdc20p depletion was obtained in cells with the sole source of Cdc20p under control of the *GAL1-10* promoter. A logarithmically growing culture in complete medium containing Raffinose and Galactose was filtered, washed with medium containing Raffinose only, and resuspended in the same medium. For release from the arrest 2% Galactose was added back to the culture.

### c) In vitro assay for Esp1p activity

A crude Triton X-100 insoluble chromatin preparation was obtained from yeast cells as described (Liang and Stillman, 1997). The pelleted chromatin was resuspended in yeast cell extracts that had been prepared similar to the supernatant fraction of the chromatin preparation. One tenth volume of an ATP regenerating system was added (50 mM HEPES/KOH pH 7.5, 100 mM KCl, 10 mM MgCl₂, 10 mM ATP, 600 mM creatin phosphate, 1.5 mg/ml phophocreatin kinase, 1 mM DTT, 10% glycerol). Reactions were incubated for 10 min at 25°C with shaking and stopped on ice. The chromatin fraction was separated again from the supernatant by centrifugation, and resuspended in buffer EBX (Liang and Stillman, 1997). Equivalent aliquots of supernatant and chromatin pellet were analysed by SDS-PAGE and Western blotting. Scc1-HA was detected with the anti-HA monoclonal antibody 16B12 (Boehringer Mannheim).

Since overexpression of Esp1p from the *GAL1-10* promoter is toxic to cells, extracts with overproduced Esp1p were prepared 2 h after induction with galactose of a culture pregrown in medium containing raffinose only.

### d) Protein sequencing of the Scc1p cleavage site

The C-terminal Scc1p cleavage fragment was isolated from cells that contained Scc1p tagged with 18 tandem myc epitopes at the C-terminus. A Cdc20 arrest/release strategy was employed to obtain cells containing a high portion of Scc1p in the cleaved form. Protein extract of 5 x 10⁹ cells was prepared by breakage with glass beads 15 min after release from the metaphase arrest. myc-epitope tagged protein was immunoprecipitated with 20 mg anti-myc 9E11 monoclonal antibody under denaturing conditions and resolved on SDS-PAGE next to size markers. Proteins were transferred to a PVDF membrane and stained with Coomassie Brillant Blue R250. N-terminal sequencing of the band corresponding to the Scc1p cleavage fragment yielded the amino acid sequence RLGESIM (Scc1p amino acids 269 onwards).

### e) Others:

Analysis of DNA content was performed as described (Epstein and Cross, 1992) on a Becton Dickinson FACScan, chromosome spreads were as described (Michaelis et al., 1997), photo micrographs were taken with a Photometrics CCD camera mounted on a Zeiss Axiophot microscope.

In vitro translation of Pds1p was performed in reticulocyte lysate using the TNT system (Promega).

### Example 1

### Chromosome association of Scc1p in G1 is Espl-dependent

A) Cells, wild type *for ESP1* or containing the *esp1-1* mutation, with an unmodified endogenous copy of Scc1 and a second myc-tagged copy under the control of the *GAL* promoter were arrested with the mating pheromone alpha factor for 120 min. All cells had then arrested in the G1 phase of the cell cycle (timepoint 0 of the experiment). The FACScan profile of the DNA content is shown, demonstrating that all cells stayed arrested during the following 120 min time course of the experiment. Scc1myc was induced for 60 min by adding 2% galactose, then cells were transferred to medium containing 2% glucose to repress Scc1myc expression (Fig. 1a).

B) Expression of Scc1myc was seen by whole cell in situ hybridization (open circles), and chromosome binding of Scc1myc was observed using chromosome spreads (filled squares). The percentage of cells positive for Scc1myc expression and that had Scc1 myc bound to chromosomes is shown in the graphs (Fig. 1b).

### Example 2

### In vitro assay for Scc1p cleavage and dissociation from chromatin

Chromatin was prepared from a strain containing Scc1p tagged with HA epitopes that was arrested in metaphase with nocodazol. The proteins in the chromatin preparation were resolved by SDS-PAGE and Scc1-HA was detected by western blotting (Fig. 2, lane 1). This chromatin preparation was resuspended in the indicated extracts, with or without addition of in vitro translation products as indicated. Incubation was for 10 min at 25°C, after which the chromatin was separated again from the supernatant by centrifugation. Aliquots of the supernatant fraction and the chromatin fraction of each reaction were analysed.

### Example 3

### Detection of the Scc1p cleavage product in vivo in cells passing sychronously throught the metaphase to anaphase transition

The strain used expressed Cdc20p under the control of the GAL promoter as the only source of Cdc20p. Scc1p was tagged with HA epitopes, and sister chromatids were visualized by tetR-GFP bound to tetO sequences inserted at the centromere of chromosome V. Cells were arrested at metaphase by depleting the cells of Cdc20p in medium lacking galactose for 120 min. Then 2% galactose was added to induce Cdc20p synthesis.
A) The FACscan profile of the timecours is shown in Fig. 3a.
B) Budding (Fig. 3b, filled squares) was scored, all cells arrested after 120 min with large buds and cytokinesis happend for most cells between 30 min and 45 min after induction of Cdc20p synthesis. Scc1-HA bound to chromosomes was seen on chromosome spreads (Fig. 3b, open circles) in most cells in the arrest, and Scc1-HA disappeared from chromosomes within 15 min after release. The percentage of cells with separated sister chromatids as seen as the occurrence of two separated GFP dots in one cell body is presented (Fig. 3b, filled triangles).
C) Examples of cells in the arrest at 120 min and 15 min after release. The synchronous separation of sister chromatids is visible as separating GFP dots (Fig. 3c).
D) Western blot analysis of whole cell extracts at the indicated time points. The cleavage fragment of Scc1-HA is apparent at 135 min short after the release from the metaphase block into anaphase (Fig. 3d).

### Example 4

Expression of a non-cleavable variant of Scc1p prevents Scc1p dissociation from chromosomes and sister chromatid separation in vivo
A) FACscan profile of the DNA content as unbudded G1 cells were released into the cell cycle either with or without the induction of the Scc1RR-DD mutant (Fig. 4a).
B) Budding index without (Fig. 4b, open squares) or with (Fig. 4b, open triangles) induction of Scc1RR-DD. Sister chromatid separation in the cells was monitored by counting the percentage of cells containing two separated GFP dots (Fig. 4b, filled squares for the control culture not expressing Scc1RR-DD, and filled triangles for the culture expressing Scc1RR-DD).
C) Scc1p chromosomse association was measured on chromosome spreads. The endogenous wild type Scc1myc is shown for the control cells (open squares) and cells expressing Scc1RR-DD (Fig. 4c, open triangles). The Scc1RR-DD was HA tagged and detected on chromosome spreads of the induced culture (Fig. 4c, filled triangles).
D) Examples of chromosome spreads of both cultures at 150 min in metaphase and at 180 min when most cells of the control culture had undergone anaphase. The DNA was stained with DAPI, Scc1myc was detected with a rabbit-anti-myc antiserum and anti-rabbit-Cy5 conjugated secondary antibody, Scc1RR-DD-HA was detected with the mouse monoclonal antibody 16B12 and anti-mouse-Cy3 conjugated secondary antibody. Sister chromatids of centromere V were visualized by the GFP dots (Fig. 4d).

### References

Ambrose WP, et al., (1998), *Anal Biochem,* Oct 15, **263**(2): 150-7

Brown AM, et al., (1994), *Anal Biochem,* Feb 15, **217**(1): 139-47

Cerretani M, et al., (1999), *Anal Biochem,* Jan 15, **266**(2): 192-7

Ciosk R, Zachariae W, Michaelis C, Shevchenko A, Mann M, and Nasmyth K (1998): An ESP1/PDS1 complex regulates loss of sister chromatid cohesion at the metaphase to anaphase transition in yeast. *Cell* **93**, 1067-1076

Cohen-Fix O, Peters J-M, Kirschner MW, and Koshland D (1996): Anaphase initiation is Sacharomyces cervisiae is controlled by the APC-dependent degradation of the anaphase inhibitor Pdslp. *Genes Dev* **10**, 3081-3093

Epstein CB, and Cross FR (1992) CLB5: a novel cyclin from budding yeast with a role in S-phase. *Genes Dev.* **6**, 1695-1706

Gietz RD and Sugino A (1988): New yeast-Escherichia coli shuttle vectors constructed with in vitro mutagenized yeast genes lacking six-base pair restriction sites. *Gene* **74**, 527-534

Gray NM, et al., (1994), *Anal Biochem,* **216**(1): 89-96

Hayden JH, Bowser SS, and Rieder CL (1990): Kinetochores capture astral microtubules during chromosome attachment to the mitotic spindle: direct visualization in live newt lung cells. *J*. *Cell Biol.* **3**, 1039-1045

Kerrebrock AW, Moore DP, Wu JS, and Orr-Weaver TL (1995): Mei-S332, a Drosophila protein required for sister chromatid cohesion, can localize to meiotic centromere regions. *Cell* **83**, 247-256

Levine LM, et al., (1997), *Anal Biochem,* Apr 5, **247**(1): 83-8

Liang C, and Stillman B (1997): Persistent initiation of DNA replication and chromatin-bound MCM proteins during the cell cycle in cdc6 mutants. *Genes Dev* **11**, 3375-3386

Lim HH, Goh P-Y, and Surana U (1998): Cdc20 is essential for the cyclosome-mediated proteolysis of both Pdsl and Clb2 during M phase in budding yeast. *Curr. Biol.* **8**, 231-234

Losada A, Hirano M, and Hirano T (1998): Identification of Xenopus SMC protein complexes required for sister chromatid cohesion. *Genes Dev* **12**, 1003-1012

McGrew JT, Goetsch L, Byers B, and Baum P (1992): Requirement for ESP1 in the nuclear division of S. cerevisiae. *Mol. Biol. Cell* **3**, 1443-1454

Merdes A, and De Mey J (1990): The mechanism of kinetochore-spindle attachment and polewards movement analyzed in PtK2 cells at the prophase-prometaphase transition. *Eur. J*. *Cell Biol.* **53**, 313-325

Michaelis C, Ciosk R, and Nasmyth K (1997): Cohesins: Chromosomal proteins that prevent premature separation of sister chromatids. *Cell* **91**, 35-45

Miyazaki WY, and Orr-Weaver TL (1994): Sister chromatid cohesion in mitosis and meiosis. *Annu. Rev. Genet.* **28**, 167-187

Moore DP, Page AW, Tang TTL, Kerrebrock AW, and Orr-Weaver TL (1998): The cohesion protein Mei-S322 localizes to condensed meiotic and mitotic centromeres until sister chromatids separate. *J*. *Cell Biol.* **140**, 1003-1012

Murray MG, et al., (1993), *Gene,* Nov 30, **134**(1):123-8

Peters J-M (1998): SCF and APC the Yin and Yan of cell cycle regulated proteolysis. *Curr. Op. Cell Biol.* **10**, 759-768

Remington's Pharmacuetical Sciences, 1980, Mack Publ. Co. Easton, PA, Osol (ed.)

Rieder CL, and Salmon ED (1998): The vertebrate cell kinetochore and its roles during mitosis. *Trends Cell. Biol.* **8**, 310-318

Rose MD, Winston F, and Hieter P (1990): Laboratory course manual for methods in Yeast Genetics (Cold Spring Harbor Laboratory Press, Cold Spring Harbor, New York)

Sarubbi E, et al., (1991), *FEBSLett,* Feb 25, **279**(2): 265-9

Singh J, et al., (1996), *BioorgMed Chem,* **4**(5): 639-43

Stebbins J. and Debouck C., (1997), *Anal Biochem,* Jun 1, **248**(2): 246-50 Taliani M, et al., (1996), *Anal Biochem,* Aug 15, **240**(1):60-7

Uhlmann F, and Nasmyth K (1998): Cohesion between sister chromatids must be established during DNA replication. *Curr. Biol.* **8**, 1095-1101

## Claims

1. Pharmaceutically active compound, characterized in that it interferes with or modulates sister chromatid separation by modulating the activity that cleaves one or more sister chromatid cohesion proteins, such as cohesin subunits.

2. The compound of claim 1, characterized in that it is a protease inhibitor specific for the proteolytic activity that cleaves one or more sister chromatid cohesion proteins.

3. Method for identifying compounds that inhibit the proliferation of rapidly dividing cells, characterized in that the protease which is responsible for the proteolytic activity involved in sister chromatid separation, optionally together with one or more co-factors, is incubated, in the presence of the substrate(s) for the protease and optionally its co-factor(s), with test compounds and that the modulating effect of the test compounds on said proteolytic activity is determined.
